# EUROPEAN PATENT APPLICATION

(11) **EP 1 967 210 A1**
(43) Date of publication of application: **10.09.2008**
(21) Application number: 06835150.1
(22) Date of filing: 19.12.2006
(51) Int. Cl.: A61K 45/00, A61K 38/00, A61P 25/18, A61P 25/20, A61P 25/22, A61P 25/24, A61P 25/28, A61P 25/30, G01N 33/15, G01N 33/50

(54) **NOVEL APPLICATION OF APELIN**

(30) Priority: 20.12.2005 JP 2005367165; 28.09.2006 JP 2006264946
(71) Applicant: Takeda Pharmaceutical Company Limited, Osaka-shi, Osaka 541-0045 (JP)
(72) Inventor: FUJII, Ryo, Osaka-shi, Osaka 532-8686 (JP)
(74) Representative: Helbing, Jörg
(86) International application number: PCT/JP2006/325703
(87) International publication number: WO 2007/072980

(57) **Abstract**

The present invention provides a preventive/therapeutic agent for mood disorders or drug dependence, and so on. A pharmaceutical composition of the present invention comprising a substance promoting the function of an apelin receptor APJ or the like is useful as the preventive/therapeutic agent for mood disorders, drug dependence, etc.

## Description

### TECHNICAL FIELD

The present invention relates to application of a substance promoting the function of an apelin receptor APJ, which is a physiologically active peptide. More particularly, the present invention relates to a pharmaceutical composition comprising the substance promoting the function of the apelin receptor APJ, a preventive/therapeutic agent for diseases, especially mood disorders, drug dependence, or the like, characterized by using the pharmaceutical composition, and so on.

### BACKGROUND ART

Mood disorders refer to mental disorders in which depressed moods continue for some time, or unusual mood elevation or abnormality lasts for a certain period of time. As generally known disease names for classification, there are bipolar disorders where recurrent periods of both manic disorder and depression occur and depression where only a depressed mood is present. On the other hand, when anxiety is long-lasting enough to interfere with daily life, the state is diagnosed as anxiety disorders. However, anxiety and depression are not as distinct from one another but considered closely associated with each other, which are frequently accompanied by physical symptoms, e.g., sleep disruption problems, anorexia, general fatigue, tiredness, etc., in addition to mental symptoms including excessive worrying, impatience, difficulty concentrating, etc. Decreased levels of serotonin, which is a brain neurotransmitter, dysfunction of noradrenaline or dopamine, abnormalities in cortisol secretion or the like are thought to be linked to causes of depression. Recently, various biological factors including hippocampal abnormalities, decreased blood flow in prefrontal cortex/anterior cingulate gyrus, etc. have also been suggested, which leads to medication-centered treatment designed to improve these factors. Some antidepressants are shown to relieve anxiety symptoms, and antidepressants (drugs used to treat depression disorders) are available for the treatment of anxiety as well. Especially, selective serotonin reuptake inhibitors are used extensively also in the treatment of anxiety. Yet existing antidepressants are known to have adverse effects such as urinary disturbances, cardiopathy, etc. before the necessary antidepressant effect is seen. It is thus desired to develop faster-acting medicaments with no side effects.

On the other hand, chemicals such as thinners, narcotics, stimulant drugs, sleeping pills, sedatives, etc. are misused for switch of mood or transient pleasure departed from their valid purposes. Consequently, the body becomes mentally and/or physically dependent on a certain drug; such a state is termed drug dependence which causes mental disorders including hallucinations or delusions. The treatment is usually centered on individual psychotherapy (counseling). While drug therapy is used to treat mental disorders such as depressive symptoms accompanied, there is no pharmacotherapy to treat drug dependence directly. It is therefore desired to develop such a pharmacotherapy as early as possible.

Apelin is a physiologically active peptide isolated as the endogenous ligand of APJ, which is one of G protein-coupled receptor proteins (WO 99/33976; Tatemoto et al., Biochemical and Biophysical Research Communications 251, 471-476 (1998)). It becomes clear that apelin binds specifically to APJ with high affinity and inhibitory G protein (Gi)-mediated intracellular signal transduction (cAMP production inhibition, etc.) is induced to exhibit a variety of physiological activities.

While it has been revealed that APJ is expressed in the mammalian brain (Hosoya, et al., Journal of Biological Chemistry 275, 21061-21067 (2000)), especially in glial cells (Medhurst et al., Journal of Neurochemistry 84, 1162-1172 (2003)), it was unknown that apelin and its receptor APJ are associated with the onset of central system disorders such as mood disorders, drug dependence, etc.

### DISCLOSURE OF INVENTION

The present invention aims at elucidating the functions of apelin and its receptor APJ in the central nervous system to provide its new use mediated by these functions.

To clarify the functions of apelin, the present inventors monitored in detail changes in expression of mRNA for APJ obtained by gene chip analysis in human brain samples from patients with various central system diseases. As a result, the inventors have found that the expression of APJ mRNA decreases in the brains from patients with depression and cocaine abusers and discovered that substances promoting the functions of apelin and APJ (e.g., agonists or the like) could be used as therapeutic agents for mood disorders, drug abuse, etc. Based on these findings, the inventors have made further investigations and come to accomplish the present invention.

That is, the present invention provides the following features, and so on.
(1) A preventive/therapeutic agent for mood disorder or drug dependence comprising a substance promoting the function of an apelin receptor.
(2) The agent according to (1) above, wherein the apelin receptor is a protein comprising the same or substantially the same amino acid sequence as the amino acid sequence represented by SEQ ID NO: 1.
(3) The agent according to (1) above, wherein the apelin receptor is a protein comprising the amino acid sequence represented by SEQ ID NO: 1.
(4) The agent according to (1) above, wherein the substance promoting the function of an apelin receptor is an agonist of the apelin receptor.
(5) The agent according to (1) above, wherein the substance promoting the function of an apelin receptor is (a) apelin, (b) an apelin derivative having an activity at least equivalent to that of apelin or (c) a low molecular synthetic compound promoting the binding of apelin and its receptor.
(6) The agent according to (5) above, wherein apelin is a polypeptide comprising the same or substantially the same amino acid sequence as the amino acid sequence represented by SEQ ID NO: 11.
(7) The agent according to (5) above, wherein apelin is a polypeptide having the amino acid sequence represented by SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24.
(8) The agent according to (5) above, wherein apelin is a polypeptide consisting of the amino acid sequence represented by SEQ ID NO: 12, SEQ ID NO: 13, SEQ ID NO: 19 or SEQ ID NO: 23.
(9) The agent according to (1) above, which is a preventive/therapeutic agent for depression or anxiety.
(10) A method for preventing/treating mood disorder or drug dependence, which comprises administering to a mammal an effective dose of a substance promoting the function of an apelin receptor.
(11) A method for preventing/treating mood disorder or drug dependence, which comprises promoting the function of an apelin receptor.
(11a) A method for preventing/treating mood disorder or drug dependence, which comprises promoting the binding of apelin and its receptor.
(12) Use of a substance promoting the function of an apelin receptor to manufacture a preventive/therapeutic agent for mood disorder or drug dependence.
(13) The method according to (10) or (11) above, wherein the apelin receptor is a protein comprising the same or substantially the same amino acid sequence as the amino acid sequence represented by SEQ ID NO: 1.
(14) The method according to (10) or (11) above, wherein the apelin receptor is a protein comprising the amino acid sequence represented by SEQ ID NO: 1.
(15) The method according to (10) or (11) above, which comprises using an agonist of an apelin receptor.
(16) The method according to (10) or (11) above, which comprises using (a) apelin, (b) an apelin derivative having an activity at least equivalent to that of apelin or (c) a low molecular synthetic compound promoting the binding of apelin and its receptor.
(17) The method according to (10) or (11) above, wherein apelin is a polypeptide comprising the same or substantially the same amino acid sequence as the amino acid sequence represented by SEQ ID NO: 11.
(18) The method according to (10) or (11) above, wherein apelin is a polypeptide having the amino acid sequence represented by SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24.
(19) The method according to (10) or (11) above, wherein apelin is a polypeptide consisting of the amino acid sequence represented by SEQ ID NO: 12, SEQ ID NO: 13, SEQ ID NO: 19 or SEQ ID NO: 23.
(19a) The method according to (10) or (11) above, which comprises using an apelin gene or an expression vector comprising the apelin gene.
(20) The method according to (10) or (11) above, which is a method for preventing/treating depression or anxiety.
(21) A method for screening of a preventive/therapeutic agent for mood disorder or drug dependence, which comprises using apelin and/or an apelin receptor.
(22) The method for screening according to (21) above, which comprises using a non-human animal deficient in gene expression of apelin.
(23) The method for screening according to (21) above, which comprises using a transgenic non-human animal carrying an exogenous gene for an apelin receptor or its mutant DNA.
(24) A kit for screening of an agent for treating mood disorder or drug dependence comprising apelin and/or an apelin receptor.

### BRIEF DESCRIPTION OF DRAWINGS

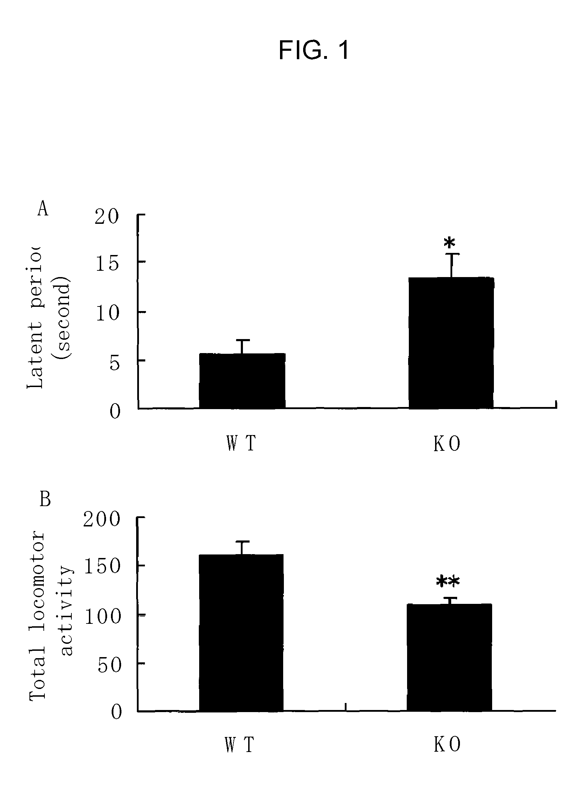

FIG. 1 shows the results of behavioral observation of wild type mice (WT) and apelin gene knockout mice (KO) in the open field test. FIG. 1A shows a time period required to completely move over from the centermost square to an adjacent square (latent period, unit in second), when the moment that mice were placed on the centermost square at the bottom of the apparatus was defined as 0 second. FIG. 1B shows the total score number (total locomotor activity) for 5 minutes from the trial start. The measurement values were all expressed as mean ± standard deviation; n=10 per group. Symbols * and ** indicate p<0.05 and p<0.01, respectively (both show comparison with wild type mice (WT)).

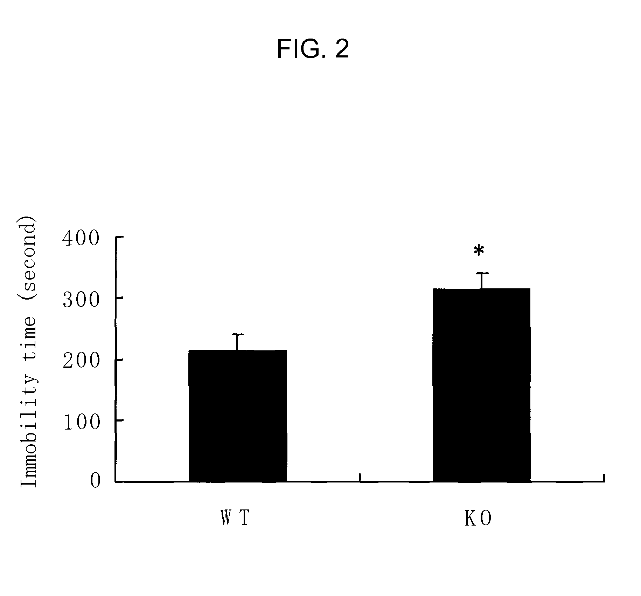

FIG. 2 shows the results of measuring the immobility time (unit in second) of wild type mice (WT) and apelin gene knockout mice (KO) according to the tail suspension test. The measurement values were all expressed as mean ± standard deviation; n=10 per group. Symbol * indicates p<0.05 (showing comparison with wild type mice (WT)).

The present invention provides pharmaceutical compositions comprising the substance promoting the function of the apelin receptor APJ (hereinafter, also referred to as the promoting agent of the present invention), preventive/therapeutic agents for diseases, especially mood disorders, drug dependence, etc. characterized by using the pharmaceutical compositions, and the like. Examples of the substance promoting the function of the apelin receptor, which constitutes the pharmaceutical composition of the present invention, include a compound having an agonist activity to the receptor, an agonist of the apelin receptor, apelin, an apelin derivative having an activity at least equivalent to that of apelin (e.g., apelin derivatives, etc. described in WO 00/18793, WO 01/70769, etc.), or a low molecular compound activating APJ, which is the apelin receptor. The substance promoting the function of the apelin receptor may be, for example, an apelin gene (DNA encoding apelin, etc.) or an expression vector comprising the apelin gene, etc. and for the same purpose as in the substance promoting the function of the apelin receptor, the substance promoting the binding of apelin and its receptor may also be used.

As used herein, "apelin" is a peptide derived from any tissue (e.g., hypophysis, pancreas, brain, kidney, liver, gonad, thyroid, gall-bladder, bone marrow, adrenal gland, skin, muscle, lung, gastrointestinal tract, blood vessel, heart, etc.) or any cell of human or warm-blooded animals (e.g., guinea pig, rat, mouse, swine, sheep, bovine, monkey, etc.) and means a peptide comprising the same or substantially the same amino acid sequence represented by, for example, SEQ ID NO: 11.

As the peptide comprising the same or substantially the same amino acid sequence as the amino acid sequence represented by SEQ ID NO: 11, preferred is a peptide comprising the same or substantially the same amino acid sequence as the amino acid sequence represented by SEQ ID NO: 11 and having the activity promoting the function of the apelin receptor. The peptide comprising the same or substantially the same amino acid sequence as the amino acid sequence represented by SEQ ID NO: 11 also includes a peptide comprising the same or substantially the same amino acid sequence as the amino acid sequence represented by SEQ ID NO: 3, SEQ ID NO: 5, SEQ ID NO: 7 or SEQ ID NO: 9, etc. That is, as used herein, the term "apelin" includes (1) a peptide comprising the same or substantially the same amino acid sequence as the amino acid sequence represented by SEQ ID NO: 3, (2) a peptide comprising the same or substantially the same amino acid sequence as the amino acid sequence represented by SEQ ID NO: 5, (3) a peptide comprising the same or substantially the same amino acid sequence as the amino acid sequence represented by SEQ ID NO: 7, (4) a peptide comprising the same or substantially the same amino acid sequence as the amino acid sequence represented by SEQ ID NO: 9, etc.

Herein, "substantially the same amino acid sequence" as the amino acid sequence represented by SEQ ID NO: 3, SEQ ID NO: 5, SEQ ID NO: 7 or SEQ ID NO: 9 includes peptides comprising (1) the amino acid sequence represented by SEQ ID NO: 3, SEQ ID NO: 5, SEQ ID NO: 7 or SEQ ID NO: 9, of which 1 to 7, preferably 1 to 5 and more preferably 1 to 3 (all inclusive) amino acids are deleted; (2) the amino acid sequence represented by SEQ ID NO: 3, SEQ ID NO: 5, SEQ ID NO: 7 or SEQ ID NO: 9, wherein 1 to 20, preferably 1 to 15 and more preferably 1 to 10 (all inclusive) amino acids are added (or inserted); (3) the amino acid sequence represented by SEQ ID NO: 3, SEQ ID NO: 5, SEQ ID NO: 7 or SEQ ID NO: 9, wherein 1 to 7, preferably 1 to 5 and more preferably 1 to 3 (all inclusive) amino acids are substituted by other amino acids; and the like.

The polypeptide comprising substantially the same amino acid sequence as the amino acid sequence represented by SEQ ID NO: 3, SEQ ID NO: 5, SEQ ID NO: 7 or SEQ ID NO: 9 includes polypeptides comprising the amino acid sequence having about 50 to 99.9% (preferably about 70 to 99.9%, more preferably about 80 to 99.9%, still more preferably about 90 to 99.9%) homology to the amino acid sequence represented by SEQ ID NO: 3, SEQ ID NO: 5, SEQ ID NO: 7 or SEQ ID NO: 9.

As the apelin to be targeted by the promoting agent of the present invention, apelin comprising the amino acid sequence represented by SEQ ID NO: 3, SEQ ID NO: 5, SEQ ID NO: 7 or SEQ ID NO: 9 (precursor) is preferred, more preferably, apelin comprising the amino acid sequence represented by SEQ ID NO: 7 (human apelin (precursor)).

The peptide comprising the same or substantially the same amino acid sequence as the amino acid sequence represented by SEQ ID NO: 11 also includes a peptide comprising the same or substantially the same amino acid sequence as the amino acid sequence represented by SEQ ID NO: 13, SEQ ID NO: 14 or SEQ ID NO: 15, etc. That is, as used herein, the term "apelin" includes (1) a peptide comprising the same or substantially the same amino acid sequence as the amino acid sequence represented by SEQ ID NO: 13, (2) a peptide comprising the same or substantially the same amino acid sequence as the amino acid sequence represented by SEQ ID NO: 14, (3) a peptide comprising the same or substantially the same amino acid sequence as the amino acid sequence represented by SEQ ID NO: 15, and the like.

Herein, "substantially the same amino acid sequence" as the amino acid sequence represented by SEQ ID NO: 13, SEQ ID NO: 14 or SEQ ID NO: 15 includes peptides comprising (1) the amino acid sequence represented by SEQ ID NO: 13, SEQ ID NO: 14 or SEQ ID NO: 15, of which 1 to 7, preferably 1 to 5, more preferably 1 to 3, much more preferably 1 to 2 (all inclusive) amino acids and most preferably 1 amino acid is deleted; (2) the amino acid sequence represented by SEQ ID NO: 13, SEQ ID NO: 14 or SEQ ID NO: 15, where 1 to 20, preferably 1 to 15, more preferably 1 to 10, still more preferably 1 to 7, much more preferably 1 to 5 and most preferably 1 to 3 (all inclusive) amino acids are added (or inserted); (3) the amino acid sequence represented by SEQ ID NO: 13, SEQ ID NO: 14 or SEQ ID NO: 15, 1 to 7, preferably 1 to 5, more preferably 1 to 3, much more preferably 1 to 2 (all inclusive) amino acids and most preferably 1 amino acid is substituted by other amino acid(s); and the like.

The polypeptide comprising substantially the same amino acid sequence as the same amino acid sequence represented by SEQ ID NO: 13, SEQ ID NO: 14 or SEQ ID NO: 15 includes polypeptides comprising the amino acid sequence having about 50 to 99.9% (preferably about 70 to 99.9%, more preferably about 80 to 99.9%, still more preferably about 90 to 99.9%, much more preferably about 93 to 99.9% and most preferably about 95 to 99.9%) homology to the amino acid sequence represented by SEQ ID NO: 13, SEQ ID NO: 14 or SEQ ID NO: 15.

As the apelin to be targeted by the promoting agent of the present invention, the apelin (apelin-36) comprising the amino acid sequence represented by SEQ ID NO: 13, SEQ ID NO: 14 or SEQ ID NO: 15 is particularly preferred, more preferably apelin comprising the amino acid sequence represented by SEQ ID NO: 13 (human apelin-36).

Preferred "apelin" also includes peptides comprising, in the 1st to 25th and 35th to 36th amino acids of the amino acid sequences represented by SEQ ID NO: 13, SEQ ID NO: 14 or SEQ ID NO: 15, (1) the amino acid sequence, of which 1 to 7, preferably 1 to 5, more preferably 1 to 3, much more preferably 1 to 2 (all inclusive) amino acids and most preferably 1 amino acid is deleted; (2) the amino acid sequence, where 1 to 20, preferably 1 to 15, more preferably 1 to 10, still more preferably 1 to 7, much more preferably 1 to 5 and most preferably 1 to 3 (all inclusive) amino acids are added (or inserted); (3) the amino acid sequence, in which 1 to 7, preferably 1 to 5, more preferably 1 to 3, much more preferably 1 to 2 (all inclusive) amino acids and most preferably 1 amino acid is substituted by other amino acid(s); and the like.

Furthermore, peptides consisting of the 2nd to 36th, 3rd to 36th, 4th to 36th, 5th to 3 6th, 6th to 3 6th, 7th to 3 6th, 8th to 3 6th, 9th to 3 6th, 10th to 3 6th, 11th to 36th, 12th to 36th, 13th to 36th, 14th to 36th, 15th to 36th, 16th to 36th, 17th to 36th, 18t to 36th, 19th to 36th, 20th to 36th, 21st to 36th (SEQ ID NO: 20), 22nd to 36th (SEQ ID NO: 19), 23rd to 36th (SEQ ID NO: 18), 24th to 36th (SEQ ID NO: 12, apelin-13), 25th to 36th (SEQ ID NO: 23), 26th to 36th (SEQ ID NO: 16), 1 st to 35th, 2nd to 35th, 3rd to 35th, 4th to 35th, 5th to 35th, 6th to 35th, 7th to 35th, 8th to 35th, 9th to 35th, 10th to 35th, 11th to 35th, 12th to 35th, 13th to 35th, 14th to 35th, 15th to 35th, 16t to 35th, 17th to 35th, 18th to 35th, 19th to 35th, 20th to 35th (SEQ ID NO: 21), 21st to 35th, 22nd to 35th, 23rd to 35th, 24th to 35th (SEQ ID NO: 24), 25th to 35th, 26th to 35th, 1st to 34th, 2nd to 34th, 3rd to 34th, 4th to 34th, 5th to 34th, 6th to 34th, 7th to 34th, 8th to 34th, 9th to 34th, 10th to 34th, 11th to 34th, 12th to 34th, 13th to 34th, 14th to 34th, 15th to 34th, 16th to 34th, 17th to 34th, 18th to 34th, 19th to 34th (SEQ ID NO: 22), 20th to 34th, 21st to 34th, 22nd to 34th, 23rd to 34th, 24th to 34th (SEQ ID NO: 17), 25th to 34th, and 26th to 34th (SEQ ID NO: 11) amino acid sequences, especially peptides consisting of the 2 1 st to 36th (SEQ ID NO: 20), 22nd to 36th SEQ ID NO: 19), 23rd to 36th (SEQ ID NO: 18), 24th to 36th (SEQ ID NO: 12, apelin-13), 25th to 36th (SEQ ID NO: 23), 26th to 36th (SEQ ID NO: 16), 20th to 35th (SEQ ID NO: 21), 24th to 35th (SEQ ID NO: 24), 19th to 34th (SEQ ID NO: 22), 24th to 34th (SEQ ID NO: 17), 26th to 34th (SEQ ID NO: 11) amino acid sequences of the SEQ ID NO: 13, SEQ ID NO: 14 or SEQ ID NO: 15 are also preferred as "apelin".

The peptide comprising the same or substantially the same amino acid sequence as the amino acid sequence represented by SEQ ID NO: 11 also includes a peptide comprising the same or substantially the same amino acid sequence as the amino acid sequence represented by SEQ ID NO: 12, etc. That is, as used herein, the "apelin" includes the peptide comprising the same or substantially the same amino acid sequence as the amino acid sequence represented by SEQ ID NO: 12, and the like.

Herein, the "substantially the same amino acid sequence" as the amino acid sequence represented by SEQ ID NO: 12 includes peptides comprising (1) the amino acid sequence represented by SEQ ID NO: 12, of which 1 to 7, preferably 1 to 5, more preferably 1 to 3, much more preferably 1 to 2 (all inclusive) amino acids and most preferably 1 amino acid is deleted; (2) the amino acid sequence represented by SEQ ID NO: 12, where 1 to 20, preferably 1 to 15, more preferably 1 to 10, still more preferably 1 to 7, much more preferably 1 to 5, particularly preferably 1 to 3 (all inclusive) amino acids and most preferably 1 amino acid is added (or inserted); (3) the amino acid sequence represented by SEQ ID NO: 12, in which 1 to 7, preferably 1 to 5, more preferably 1 to 3 and much more preferably 1 to 2 (all inclusive) amino acids and most preferably 1 amino acid is substituted by other amino acid(s); and the like.

The polypeptide comprising the substantially the same amino acid sequence as the amino acid sequence represented by SEQ ID NO: 12 includes polypeptides comprising an amino acid sequence having about 50 to 99.9% (preferably about 70 to 99.9%, more preferably about 80 to 99.9%, still more preferably about 90 to 99.9%, much more preferably about 93 to 99.9%) homology to the amino acid sequence represented by SEQ ID NO: 12.

As the apelin to be targeted by the promoting agent of the present invention, the apelin comprising the amino acid sequence represented by SEQ ID NO: 12 (apelin-13) is particularly preferred.

Preferred "apelin" also includes peptides comprising, in the 1st to 2nd and 12th to 13th amino acids of the amino acid sequence represented by SEQ ID NO: 12, (1) the amino acid sequence, of which, 1 to 4, preferably 1 to 3, more preferably 1 to 2 (all inclusive) amino acids and most preferably 1 amino acid is deleted; (2) the amino acid sequence, where 1 to 20, preferably 1 to 15, more preferably 1 to 10, still more preferably 1 to 7, much more preferably 1 to 5 and most preferably 1 to 3 (all inclusive) amino acids are added (or inserted); (3) the amino acid sequence, in which 1 to 4, preferably 1 to 3, more preferably 1 to 2 (all inclusive) amino acids and most preferably 1 amino acid is substituted by other amino acid(s); and the like.

Preferred "apelin" also includes peptides consisting of the 2nd to 13th amino acid sequence (SEQ ID NO: 23), 3rd to 13th (SEQ ID NO: 16), 1st to 12th (SEQ ID NO: 24), 2nd to 12th, 3rd to 12th, 1st to 11th (SEQ ID NO: 17), 2nd to 11th, 3rd to 11th (SEQ ID NO: 11) amino acid sequences, especially, peptides consisting of the 2nd to 13th (SEQ ID NO: 23), 3rd to 13th (SEQ ID NO: 16), 1 st to 12th (SEQ ID NO: 24), 1st to 11th (SEQ ID NO: 17), 3rd to 11th (SEQ ID NO: 11) amino acid sequences of SEQ ID NO: 12.

The peptide comprising the same or substantially the same amino acid sequence as the amino acid sequence represented by SEQ ID NO: 11 includes peptides consisting of the 42nd to 77th (SEQ ID NO: 13, SEQ ID NO: 14 or SEQ ID NO: 15), 43rd to 77th, 44th to 77th, 45th to 77th, 46th to 77th, 47th to 77th, 48th to 77th, 49th to 77th, 50th to 77th, 51st to 77th, 52nd to 77th, 53rd to 77th, 54th to 77th, 55th to 77th, 56th to 77th, 57th to 77th, 58th to 77th, 59th to 77th, 60th to 77th, 61st to 77th, 62nd to 77th (SEQ ID NO: 20), 63rd to 77th (SEQ ID NO: 19), 64th to 77th (SEQ ID NO: 18), 65th to 77th (SEQ ID NO: 12, apelin-13), 66th to 77th (SEQ ID NO: 23), 67th to 77th (SEQ ID NO: 16), 42nd to 76th, 43rd to 76th, 44th to 76th, 45th to 76th, 46th to 76th, 47th to 76th, 48th to 76th, 49th to 76th, 50th to 76th, 51st to 76th, 52nd to 76th, 53rd to 76th, 54th to 76th, 55th to 76th, 56th to 76th, 57th to 76th, 58th to 76th, 59th to 76th, 60th to 76th, 61st to 76th (SEQ ID NO: 21), 62nd to 76th, 63rd to 76th, 64th to 76th, 65th to 76th (SEQ ID NO: 24), 66th to 76th, 67th to 76th, 42nd to 75th, 43rd to 75th, 44th to 75th, 45th to 75th, 46th to 75th, 47th to 75th, 48th to 75th, 49th to 75th, 50th to 75th, 51st to 75th, 52nd to 75th, 53rd to 75th, 54th to 75th, 55th to 75th, 56th to 75th, 57th to 75th, 58th to 75th, 59th to 75th, 60th to 75th (SEQ ID NO: 22), 6 1 st to 75th, 62nd to 75th, 63rd to 75th, 64th to 75th, 65th to 75th (SEQ ID NO: 17), 66th to 75th, and 67th to 75th (SEQ ID NO: 11) amino acid sequences in the amino acid sequence of SEQ ID NO: 3, SEQ ID NO: 5, SEQ ID NO: 7 or SEQ ID NO: 9. Preferred examples of the peptide comprising the same or substantially the same amino acid sequence as the amino acid sequence represented by SEQ ID NO: 11, the peptide consisting of the 42nd to 77th (SEQ ID NO: 14) amino acid sequences of SEQ ID NO: 3; 42nd to 77th (SEQ ID NO: 13) amino acid sequences of SEQ ID NO: 7; 42nd to 77th (SEQ ID NO: 15) amino acid sequence of SEQ ID NO: 9; 62nd to 77th (SEQ ID NO: 20), 63rd to 77th (SEQ ID NO: 19), 64th to 77th (SEQ ID NO: 18), 65th to 77th (SEQ ID NO: 12, apein-13), 66th to 77th (SEQ ID NO: 23), 67th to 77th (SEQ ID NO: 16), 61st to 76th (SEQ ID NO: 21), 65th to 76th (SEQ ID NO: 24), 60th to 75th (SEQ ID NO: 22), 65th to 75th (SEQ ID NO: 17) and 67th to 75th (SEQ ID NO: 11) amino acid sequences of SEQ ID NO: 3, SEQ ID NO: 5, SEQ ID NO: 7 or SEQ ID NO: 9.

As the apelin receptor (APJ) to be targeted by the promoting agent of the present invention, a protein comprising the same or substantially the same amino acid sequence as the amino acid sequence represented by SEQ ID NO: 1 is preferred. Herein, the term "substantially the same" is used to mean that the amino acid has homology of about 50 to 99.9% (preferably about 70 to 99.9%, more preferably about 80 to 99.9%, still more preferably about 90 to 99.9%) to the amino acid sequence represented by SEQ ID NO: 1.

Furthermore, "substantially the same amino acid sequence" as the amino acid sequence represented by SEQ ID NO: 1 includes peptides comprising (1) the amino acid sequence represented by SEQ ID NO: 1, of which 1 to 7, preferably 1 to 5 and more preferably 1 to 3 (all inclusive) amino acids are deleted; (2) the amino acid sequence represented by SEQ ID NO: 1, wherein 1 to 20, preferably 1 to 15 and more preferably 1 to 10 (all inclusive) amino acids are added (or inserted); (3) the amino acid sequence represented by SEQ ID NO: 1, wherein 1 to 7, preferably 1 to 5 and more preferably 1 to 3 (all inclusive) amino acids are substituted by other amino acids; and the like.

Throughout the specification, the polypeptides are represented in accordance with the conventional way of describing peptides, that is, the N-terminus (amino terminus) at the left hand and the C-terminus (carboxyl terminus) at the right hand. In the polypeptides of the present invention, the C-terminus may be in the form of a carboxyl group (-COOH), a carboxylate (-COO⁻), an amide (-CONH₂) or an ester (-COOR), or in the form of a salt. Herein, examples of the ester group represented by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; an α-naphthyl-C₁₋₂ alkyl group such as α-naphthylmethyl, etc.; and the like. In addition, pivaloyloxymethyl or the like, which is used widely as an ester for oral administration, may also be used. Where the polypeptide of the present invention contains a carboxyl group or a carboxylate at a site other than the C-terminus, the polypeptide of the present invention also includes those wherein these groups are amidated or esterified. Esters used in this case are, for example, the C-terminal esters described above. The polypeptide of the present invention also includes those wherein the N-terminal region of Gln is cleaved in vivo and this Gln is pyroglutaminated, and the like.

In addition, the polypeptide of the present invention further includes those wherein Met is added to the N-terminus. These polypeptides may also be partial peptides thereof.

As salts of the polypeptides of the present invention, salts with physiologically acceptable bases (e.g., alkali metal salts) or acids (e.g., inorganic acids or organic acids) may be employed, preferably in the form of physiologically acceptable acid addition salts. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid and benzenesulfonic acid) and the like.

The polypeptide of the present invention may be manufactured from human or other warm-blooded animal cells or tissues by a publicly known method of purifying a polypeptide, or may be manufactured by modifications of the polypeptide synthesis methods later described. They may also be manufactured by culturing a transformant containing a DNA encoding the polypeptide, as will be later described.

Where the polypeptide is manufactured from human or warm-blooded animal tissues or cells, human or warm-blooded animal tissues or cells are homogenized, then extracted with an acid, etc. and the extract is isolated and purified by a combination of chromatography techniques such as reverse phase chromatography, ion exchange chromatography, and the like.

The polypeptide of the present invention can be manufactured by publicly known methods for polypeptide synthesis or by cleaving the polypeptide comprising the polypeptide of the present invention with an appropriate peptidase. For the methods for peptide synthesis, for example, either solid phase synthesis or liquid phase synthesis may be used. That is, the partial peptide or amino acids that can construct the polypeptide of the present invention are condensed with the remaining part. Where the product contains protecting groups, these protecting groups are removed so that the desired peptide can be manufactured. Publicly known methods for condensation and elimination of the protecting groups are described in (a) - (e) below.
(a) M. Bodanszky & M.A. Ondetti: Peptide Synthesis, Interscience Publishers, New York (1966)
(b) Schroeder & Luebke: The Peptide, Academic Press, New York (1965)
(c) Nobuo Izumiya, et al.: Peptide Gosei-no-Kiso to Jikken (Basics and experiments of peptide synthesis), published by Maruzen Co. (1975)
(d) Haruaki Yajima & Shunpei Sakakibara: Seikagaku Jikken Koza (Biochemical Experiment) 1, Tanpakushitsu no Kagaku (Chemistry of Proteins) IV, 205 (1977)
(e) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the polypeptide of in the present invention. When the polypeptide obtained by the above methods is in a free form, the polypeptide can be converted into an appropriate salt by a publicly known method; conversely when the polypeptide is obtained in a salt form, it can be converted into a free form by a publicly known method.

To synthesize amides of the polypeptide of the present invention, commercially available resins suitable for amide formation may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin, etc. Using these resins, amino acids in which α-amino groups and functional groups on the side chains are appropriately protected are condensed on the resin in the order of the sequence of the objective peptide according to various condensation methods publicly known in the art. At the end of the reaction, the peptide is excised from the resin and at the same time, the protecting groups are removed. Then, intramolecular disulfide bond-forming reaction is performed in a highly diluted solution to obtain the objective polypeptide.

For condensation of the protected amino acids described above, a variety of activation reagents for peptide synthesis may be used, particularly preferably carbodiimides. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt, etc.) are added directly to the resin, or the protected amino acids are previously activated in the form of symmetric acid anhydrides, HOBt esters or HOOBt esters, followed by adding the thus activated protected amino acids to the resin. Solvents suitable for use to activate the protected amino acids or condense with the resin may be chosen from solvents that are known to be usable for peptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the amino groups in the starting amino acids include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc. Examples of the protecting groups for carboxyl groups include, in addition to a C₁₋₆ alkyl group, a C₃₋₈ cycloalkyl group and a C₇₋₁₄ aralkyl group, 2-adamantyl, 4-nitrobenzyl, 4-methoxybenzyl, 4-chlorobenzyl, phenacyl and benzyloxycarbonylhydrazide, tertiary-butoxycarbonylhydrazide, tritylhydrazide, etc.

The hydroxyl group of serine and threonine can be protected through, for example, esterification or etherification. Examples of groups appropriately used for the esterification include a lower alkanoyl group such as acetyl, etc., an aroyl group such as benzoyl group, and a group derived from carbon such as benzyloxycarbonyl group, ethoxycarbonyl group, etc. Examples of groups appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, tertiary-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids in which the amino groups are activated in the starting material, for example, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. Elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups and activation, etc. of functional groups involved in the reaction may be appropriately selected from publicly known groups or publicly known means.

In another method for obtaining the amides of the polypeptide, for example, the α-carboxyl group of the carboxy terminal amino acid is first protected by amidation; the peptide chain is then extended from the amino group side to a desired length. Thereafter, a peptide (or an amino acid) in which only the protecting group of the N-terminal α-amino group in the peptide chain is eliminated and a peptide in which only the protecting group of the C-terminal carboxyl group is eliminated are manufactured. The two peptides are condensed in a mixture of the solvents described above. The details of the condensation reaction are the same as described above. After the protected peptide obtained by the condensation is purified, all the protecting groups are removed by the method described above to give the desired crude polypeptide. This crude polypeptide is purified by various known purification means. Lyophilization of the major fraction gives the amide of the desired polypeptide.

To prepare the esterified polypeptide, for example, the α-carboxyl group of the carboxy terminal amino acid is condensed with a desired alcohol to prepare the amino acid ester, which is followed by a procedure similar to the preparation of the amidated polypeptide to give the desired esterified polypeptide.

The polypeptide of the present invention may be any peptide as long as it exhibits similar activities (e.g., vascular endothelial cell migration, growth inhibition activity, angiogenesis inhibitory action, etc.) to those described above. These peptides include, for example, peptides comprising a partial sequence of the amino acid sequence represented by SEQ ID NO: 3, SEQ ID NO: 5, SEQ ID NO: 7 or SEQ ID NO: 9, in which at least 1 amino acid is deleted, etc. Specifically, preferred are peptides having a partial sequence of the amino acid sequence represented by SEQ ID NO: 3, SEQ ID NO: 5, SEQ ID NO: 7 or SEQ ID NO: 9.

The polypeptide of the present invention may also be a fusion protein with a protein which function or property is well known.

The polypeptide of the present invention and a DNA encoding the polypeptide of the present invention described later may be labeled by a public known method, and specifically includes those labeled with an isotope, those labeled with a fluorescence (e.g., fluorescence labeling with fluorescein), those biotinylated, those labeled with an enzyme, etc.

The DNA encoding the polypeptide of the present invention (in particular, the apelin receptor) may be any DNA so long as it contains a nucleotide sequence encoding the polypeptide having the binding activity to a receptor protein comprising the same or substantially the same amino acid sequence as the amino acid sequence represented by SEQ ID NO: 1. Specifically, the DNA may be any DNA so long as it contains a nucleotide sequence encoding the polypeptide comprising the same or substantially the same amino acid sequence as the amino acid sequence of the polypeptide of the present invention. The DNA may also be any one of genomic DNA, genomic DNA library, cDNA derived from the tissues or cells described above, cDNA library derived from the tissues or cells described above and synthetic DNA. The vector to be used for the library may be any of bacteriophage, plasmid, cosmid, phagemid, and the like. The DNA can also be directly amplified by reverse transcriptase polymerase chain reaction (hereinafter abbreviated as RT-PCR) using the RNA fraction prepared from the cells or tissues described above.

Examples of the DNA encoding the polypeptide of the present invention may be any one of (1) a DNA comprising a DNA having the entire or part of a nucleotide sequence represented by SEQ ID NO: 4, SEQ ID NO: 6, SEQ ID NO: 8 or SEQ ID NO: 10, (2) a mammal-derived DNA that is hybridizable to the sequence defined in (1) under stringent conditions, (3) a DNA that is not hybridizable to the sequence defined in (1) and (2) due to degeneracy of genetic code but encodes the polypeptide having the same amino acid sequence, etc. The hybridization can be carried out by publicly known methods or by modifications of such known methods. The stringent conditions described above are, for example, conditions of 42°C, 50% formamide, 4 x SSPE (1 x SSPE = 150 mM NaCl, 10 mM NaH₂PO₄.H₂O, 1 mM EDTA, pH 7.4), 5 x Denhardt's solution and 0.1% SDS.

The DNA encoding the polypeptide of the present invention can also be manufactured by the following genetic engineering techniques.

Means for cloning the DNA which completely encodes the polypeptide of the present invention includes (1) a method which involves amplifying the objective DNA from the DNA library, etc. described above by publicly known PCR using synthetic DNA primers having a part of the nucleotide sequence of the polypeptide of the present invention, or (2) a method which involves inserting a DNA into an appropriate vector and selecting the DNA, for example, by hybridization with a labeled DNA fragment or synthetic DNA that encodes a part or entire region of the polypeptide of the present invention. The hybridization can be carried out, for example, according to the method described in Molecular Cloning, 2nd (J. Sambrook et al., Cold Spring Harbor Lab. Press, 1989), etc. When a commercially available library is used, the hybridization is performed in accordance with the protocol described in the attached instructions.

The cloned DNA encoding the polypeptide of the present invention can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof these translation initiation and termination codons may also be added by using an appropriate synthetic DNA adapter.

The expression vector for the polypeptide of the present invention can be manufactured, for example, by (a) excising the desired DNA fragment from the DNA encoding the polypeptide of the present invention, and then (b) ligating the DNA fragment with an appropriate expression vector downstream a promoter in the vector.

Examples of the vector include plasmids derived form E. coli (e.g., pBR322, pBR325, pUC12 or pUC13), plasmids derived from Bacillus subtilis (e.g., pUB110, pTP5 or pC194), plasmids derived from yeast (e.g., pSH19 or pSH15), bacteriophages such as λ phage, etc., animal viruses such as retrovirus, vaccinia virus, baculovirus, etc.

The promoter used in the present invention may be any promoter if it matches well with a host to be used for gene expression.

In the case of using animal cells as the host, examples of the promoter include SRα promoter, SV40 promoter, LTR promoter, CMV promoter, HSV-TK promoter, etc. Among them, CMV promoter or SRα promoter is preferably used. Where the host is bacteria of the genus Escherichia, preferred examples of the promoter include trp promoter, lac promoter, recA promoter, λPL promoter, lpp promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary, a signal sequence that matches a host is added to the N-terminus of the polypeptide. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector bearing the DNA encoding the polypeptide thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)), JM103 (Nucleic Acids Research, 9, 309 (1981)], JA221 [Journal of Molecular Biology, 120, 517 (1978)], HB101 [Journal of Molecular Biology, 41, 459 (1969)), C600 (Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, etc.

As the insect, for example, a larva of Bombyx mori, etc. can be used [Maeda, et al., Nature, 315, 592 (1985)].

Examples of insect cells include, for the virus AcNPV, Spodoptera frugiperda cells (Sf cells), MG1 cells derived from mid-intestine of Trichoplusia ni, High Five^{™} cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells [both cells are described in Vaughn, J. L. et al., In Vivo, 13, 213-217 (1977)], etc.

Examples of animal cells include monkey cells COS-7, Vero cells, Chinese hamster cells CHO, DHFR gene-deficient Chinese hamster cells CHO (dhfr⁻-CHO cells), mouse L cells, mouse 3T3 cells, mouse myeloma cells, human HEK293 cells, human FL cells, 293 cells, C127 cells, BALB3T3 cells, Sp-2/O cells, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Virology, 52, 456(1973).

Methods for introducing the expression vectors into the cells include, for example, the lipofection method [Felgner,P.L. et al., Proceedings of the National Academy of Sciences of the United States of America, 84, 7413 (1987)], the calcium phosphate method [Graham,F.L. and van der Eb, A.J., Virology, 52, 456-467 (1973)], the electroporation method [Nuemann,E. et al., EMBO J., 1, 841-845 (1982)], etc.

As described above, the transformant transformed by the expression vector bearing the DNA encoding the polypeptide of the present invention can be obtained.

Methods for stably expressing the polypeptide of the present invention using animal cells include methods of selecting the cells by clone selection in which the expression vectors described above are introduced into chromosomes. Specifically, transformants can be selected based on the selection markers described above. Further, repeated clone selections on the transformants thus obtained using the selection markers enable to acquire stable animal cell lines capable of highly expressing the polypeptide of the present invention. Furthermore, when the dhfr gene is used as the selection marker, incubation may be carried out by gradually increasing the concentration of MTX to select resistant cells, whereby the DNA encoding the polypeptide of the present invention is amplified in the cells concurrently with the dhfr gene to acquire animal cell lines with higher expression.

The transformants described above are cultured under conditions capable of expressing the DNA encoding t the polypeptide of the present invention to produce and accumulate the polypeptide of the present invention, whereby the polypeptide of the present invention can be produced.

Where the host is bacteria belonging to the genus Escherichia or the genus Bacillus, the transformant can be appropriately cultured in a liquid medium, which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

Especially when CHO (dhfr⁻) cells and dhfr gene are used as selection markers, it is preferred to use substantially thymidine-free DMEM medium supplemented with dialyzed fetal calf serum.

The polypeptide of the present invention can be separated and purified from the culture described above, e.g., by the procedures described below.

When the polypeptide of the present invention is extracted from the culture or cells, after incubation the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the polypeptide of the present invention can be obtained. The buffer used for the procedures may contain a protein modifier such as urea or guanidine hydrochloride, or a surfactant such as Triton (registered trademark) X-100, etc.

When the polypeptide of the present invention is released in the culture, after completion of the incubation the supernatant can be separated from the transformants or cells to collect the supernatant by a publicly known method.

The polypeptide of the present invention contained in the culture supernatant or the extract thus obtained can be purified by appropriately combining publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis, chromatofocusing, etc.; and the like.

Where the polypeptide of the present invention obtained by the above methods is in a free form, it can be converted into an appropriate salt by a publicly known method or its modifications; where the polypeptide is obtained in a salt form, it can be converted into a free form or into the other salt by a publicly known method or its modifications.

The polypeptide of the present invention produced by the recombinant can be treated, prior to or after the purification, with an appropriate protein modifying enzyme to appropriately modify the same or partially remove a polypeptide. Examples of the protein-modifying enzyme include trypsin, chymotrypsin, arginyl endopeptidase, protein kinase, glycosidase, or the like.

The presence of the polypeptide of the present invention thus produced can be assayed by enzyme immunoassay, etc. using a specific antibody, or the like.

Using the apelin and/or its receptor obtained as described above, the substance which promotes their binding can be produced as follows.

The substance which promotes the binding of apelin and its receptor provided by the present invention, and the agonist of the apelin receptor as the substance which promotes the function of the apelin receptor (the agonist of the present invention) will be described below in detail.

Examples of the agonist of the present invention include peptides, proteins, antibodies, non-peptide compounds, synthetic compounds, fermentation products, cell extracts, plant extracts, animal tissue extracts, blood plasma, etc. These substances may be either novel substances or publicly known substances. The agonist of the present invention may form salts, and these salts of the agonist used are the same as those of the polypeptide of the present invention given above.

Since the agonist of the present invention can promote the function of the apelin receptor, the agonist can be screened using apelin and its receptor. The method of screening is described below.

The agonist of the present invention can be obtained using the method screening the substance promoting the function of the apelin receptor, which comprises comparing (i) the case where apelin is brought in contact with the apelin receptor and (ii) the case where a test compound is brought in contact with the apelin receptor. In the screening method of the present invention, for example, (a) the binding amounts of apelin to the apelin receptor are measured (i) when apelin is brought in contact with the apelin receptor and (ii) apelin and a test compound are brought in contact with the apelin receptor; and comparing the results; or, (b) cell stimulating activities (e.g., the activities that promote arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH changes, etc.) mediated by the apelin receptor are measured (i) when apelin is brought in contact with the apelin receptor and (ii) a test compound is brought in contact with the apelin receptor; and comparing the results.

Specific examples of the screening method of the present invention include:
(1) a method of screening the substance promoting the function of the apelin receptor, which comprises measuring the binding amounts of labeled apelin to the apelin receptor when the labeled apelin is brought in contact with the apelin receptor and when the labeled apelin and a test compound are brought in contact with the apelin receptor; and comparing the amounts;
(2) a method of screening the substance promoting the function of the apelin receptor, which comprises measuring the binding amounts of labeled apelin to a cell containing the apelin receptor or a membrane fraction of the cell, when the labeled apelin is brought in contact with the cell or membrane fraction and when the labeled apelin and a test compound are brought in contact with the cell or membrane fraction, and comparing the binding amounts; and,
(3) a method of screening the substance promoting the function of the apelin receptor, which comprises measuring the binding amounts of labeled apelin to the apelin receptor expressed on a cell membrane by culturing a transformant having a DNA encoding the apelin receptor, when the labeled apelin is brought in contact with the apelin receptor and when the labeled apelin and a test compound are brought in contact with the apelin receptor, and comparing the binding amounts, and the like.

Specifically, the screening of the substance promoting the binding of apelin and the apelin receptor can also be performed by the following procedures. First, a standard receptor sample is produced by suspending cells containing the apelin receptor or their membrane fractions in a buffer appropriate for screening. Any buffer can be used so long as it does not interfere with the binding of apelin and the apelin receptor, such buffer including a phosphate buffer, a Tris-HCl buffer, etc. having pH of 4 to 10 (desirably pH of 6 to 8). For the purpose of minimizing non-specific binding, a surfactant such as CHAPS, Tween-80^{™} (manufactured by Kao-Atlas Inc.), digitonin, deoxycholate, etc. may be added to the buffer. Further for the purpose of suppressing degradation of the apelin receptor or apelin by a protease, a protease inhibitor such as PMSF, leupeptin, E-64 (manufactured by Peptide Institute, Inc.), pepstatin, etc. may also be added. A given quantity (5,000 cpm to 500,000 cpm) of labeled apelin or its modified form is added to 0.01 ml to 10 ml of a solution of the apelin receptor, and at the same time, 10⁻⁴ to 10⁻¹ µM of a test compound is allowed to be co-present. To determine the amount of non-specific binding (NSB), a reaction tube containing a large excess of unlabeled apelin is also provided. The reaction is carried out at about 0°C to about 50°C, preferably about 4°C to about 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or a γ-counter. When the nonspecific binding (NSB) is subtracted from the count (B₀) when any antagonizing compound is absent and the thus obtained count (B₀ - NSB) is made 100%, a test compound having the specific binding (B - NSB) of, e.g., 100% or more, can be selected as a candidate substance.

Examples of the test compound include peptides, proteins, antibodies, non-peptide compounds, synthetic compounds, fermentation products, cell extracts, plant extracts, animal tissue extracts, blood plasma, etc. These substances may be either novel substances or publicly known substances. The test compound may form salts, and these salts of the test compound used are the same as those of the polypeptide of the present invention given above.

The agonist thus obtained can promote the physiological activities possessed by apelin and can thus be used as a safe and low-toxic medicament.

### [Pharmaceutical composition comprising the agonist of the present invention]

The pharmaceutical composition comprising the agonist of the present invention which is produced based on the description above can be manufactured and used in a conventional manner. For example, the composition can be administered orally in the form of tablets which may be sugar coated or enteric coated, if necessary, capsules, elixirs, microcapsules, sustained release formulations, etc., or parenterally in the form of injections such as sterile solutions or suspensions or sustained release formulations in water or in pharmaceutically acceptable solutions other than water. For example, the pharmaceutical composition can be mixed with carriers, flavoring agents, excipients, vehicles, preservatives, stabilizers, binders, etc. in a unit dosage form generally accepted. The active ingredient in these preparations is controlled in such a dose that an appropriate dose is obtained within the specified range given. The agonist of the present invention is used alone, but when it is used as a composition, 10% to 90% of the agonist is formulated in the composition as the active ingredient.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated, e.g., by dissolving or suspending the active ingredients in a vehicle such as water for injection with a naturally occurring vegetable oil such as sesame oil and coconut oil, etc. in a conventional manner to prepare the pharmaceutical preparations.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80^{™} and HCO-50), etc. Examples of the oily medium include sesame oil and soybean oil, which may also be used in combination with a dissolution aid such as benzyl benzoate, benzyl alcohol, etc.

The injection may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The injection thus prepared is usually filled in an appropriate ampule. Since the pharmaceutical preparation thus obtained is safe and low toxic, it can be administered to human or mammals (e.g., mice, rats, guinea pigs, rabbits, sheep, swine, bovine, cats, dogs, monkeys, chimpanzee, etc.).

The dose of the pharmaceutical composition of the present invention varies depending on conditions, etc.; in oral administration, the active ingredient (that is, the polypeptide of the present invention, its amide or ester, or a salt thereof) is administered to an adult patient (as 60 kg body weight) with bipolar disorder normally at a daily dose of about 0.1 mg to about 100 mg, preferably about 1.0 to about 50 mg, and more preferably about 1.0 to about 20 mg. In parenteral administration, its single dose varies depending on subject to be administered, target organs, conditions, route for administration, etc. When the pharmaceutical composition is administered to a patient (as 60 kg body weight) with bipolar disorder in the form of an injectable preparation, it is advantageous to intravenously administer the active ingredient (that is, the polypeptide of the present invention, its amide or ester, or a salt thereof) at a daily dose of about 0.01 to about 30 mg, preferably about 0.1 to about 20 mg, and more preferably about 0.1 to about 10 mg. For other animal species, the corresponding dose as converted per 60 kg body weight can be administered.

The substance of the present invention which promotes the function of apelin and its receptor, including apelin, apelin derivatives having activities equivalent to those of apelin, low molecular compounds promoting the binding of apelin and its receptor, etc., or the substance which activates the apelin receptor APJ can be used as safe and low toxic preventive/therapeutic agents for, e.g., mood disorders and drug dependence, or their adjuncts.

Examples of mood disorders are bipolar disorders, depression, manic disorder, recurrent depression, persistent mood affective disorders (e.g., cyclothymia, dysthymia, etc.), depressive neurosis, anxiety (e.g., generalized anxiety disorders, social anxiety disorders, obsessive-compulsive disorders, panic disorders, posttraumatic stress disorders, etc.), sleeping disorders, eating disorders, autism, senile dementia, schizophrenia, attention deficit hyperactivity disorders, psychosomatic disorders, etc.

### [Animal deficient in apelin gene expression, APJ gene transgenic animal]

The non-human animal deficient in apelin gene expression of the present invention (hereinafter, sometimes referred to as the non-human animal deficient in gene expression) is used to mean, for example, an animal produced by genetic engineering using mammalian ES cell-derived cells in which the aforesaid apelin gene is inactivated, e.g., a non-human animal where a sequence of inactivated apelin gene is introduced into the germ cells and somatic cells at early stages of embryogenesis.

Any animal other than a human carrying apelin may be used as the non-human animal, preferably a non-human mammal. As the non-human mammals, for example, bovine, swine, sheep, goats, rabbits, dogs, cats, guinea pigs, hamsters, mice, rats, etc. are used. Among them, preferred are rodents, especially mice (e.g., C57BL/6 strain, DBA2 strain, etc. for a pure line and for a cross line, B6C3F₁ strain, BDF₁ strain, B6D2F₁ strain, BALB/c strain, ICR strain, etc.) (among others, preferably C57BL/6 strain, etc. for the pure line and BDF1 strain, ICR strain, etc. for the cross line) or rats (e.g., Wistar, SD, etc.), because these animals are relatively short in ontogeny and life cycle from a standpoint of preparing model animals for human diseases.

For knockout of the apelin gene, the targeting vector described above is introduced into non-human mammalian ES cells or non-human mammalian oocytes by public known methods (e.g., electroporation, microinjection, calcium phosphate, lipofection, agglutination, particle gun and DEAE-dextran methods, etc.) (preferred methods for introduction are electroporation when introduced into ES cells and microinjection when introduced into oocytes, etc.), followed by exchange of the sequence of inactivated apelin gene of the targeting vector with the apelin gene on the chromosomes of non-human animal ES cells or non-human animal oocytes by homologous recombination.

The apelin gene-knockout cells can be assessed by the Southern hybridization analysis using as a probe a DNA sequence on or near the apelin gene, or by the PCR analysis using as primers a DNA sequence on the targeting vector and another DNA sequence at the proximal region other than the mouse-derived apelin gene used as the targeting vector.

When non-human mammalian ES cells are used, a cell line wherein the apelin gene is inactivated by homologous recombination is cloned, and the clones are injected into, e.g., a non-human mammalian embryo or blastocyst at an appropriate stage such as the 8-cell stage (injection method), or ES cell clusters wherein the apelin gene is inactivated are inserted into two 8-cell stage embryos (aggregation chimera method). The chimeric embryos thus prepared are transplanted to the uterus of the pseudopregnant non-human mammal.

The animal thus created is a chimeric animal composed of both cells having a normal locus of the apelin gene and cells having an artificially mutated locus of the apelin gene.

When some germ cells of the chimeric animal have a mutated locus of the apelin gene, an individual, which entire tissue is composed of cells having a mutated locus of the apelin gene, can be selected from a series of offsprings obtained by crossing between such a chimeric animal and a normal animal, e.g., by coat color identification, etc. The individuals thus obtained are normally deficient in heterozygous apelin expression. The individuals deficient in homozygous apelin expression can be obtained from offsprings of the intercross between those deficient in heterozygous apelin or APJ expression.

The non-human animal deficient in apelin gene expression can be identified distinctly from normal animals by measuring mRNA levels of the animal and indirectly comparing the expression levels using a public known method.

As described above, the individuals in which the apelin gene is rendered knockout permit the passage rearing under ordinary rearing conditions, after the individuals obtained by their crossing are proven to be knockout.

Also, the genital system can be acquired and retained by conventional methods. That is, by crossing male and female animals each having the inactivated gene sequence, homozygous animals having the inactivated gene sequence in both loci can be obtained. The homozygotes thus obtained can be reared so that one normal animal and two or more homozygotes are produced from a mother animal to obtain such homozygotes efficiently. By crossing male and female heterozygotes, homozygotes and heterozygotes having the inactivated gene sequence can be proliferated and passaged. The offsprings of the thus obtained animal having the inactivated gene sequence are also included in the non-human animal deficient in apelin gene expression of the present invention.

The mammalian ES cells, in which the apelin gene is inactivated, are very useful for preparing a non-human animal deficient in apelin gene expression. Since the non-human animal deficient in apelin gene expression or its tissue or cells derived from the animal can be a better disease model suspected of diseases caused by apelin deficiency, for example, diseases caused by inactivated biological activities of apelin based on lacks of various apelin-induced biological activities (e.g., bipolar disorders, depression, mania, recurrent depression, persistent mood affective disorders (e.g., cyclothymia, dysthymia, etc.), depressive neurosis, anxiety (e.g., generalized anxiety disorders, social anxiety disorders, obsessive-compulsive disorders, panic disorders, posttraumatic stress disorders, etc.), sleeping disorders, eating disorders, autism, senile dementia, schizophrenia, attention deficit hyperactivity disorders, psychosomatic disorders, etc.; drug dependency, etc.). Therefore, the animal is effective for the study to investigate the causes and therapy for these diseases.

As described above, the non-human animal deficient in apelin gene expression of the present invention, or its tissue or cells derived from the same can be used for screening of the preventing and/or therapeutic agent for the diseases. Herein, examples of the tissues and cells derived therefrom include hypophysis, pancreas, brain, kidney, liver, gonad, thyroid, gall-bladder, bone marrow, adrenal gland, skin, muscle, lung, gastrointestinal tract, blood vessel, heart, etc., or cells derived therefrom, etc. The non-human animal deficient in expression of the gene of the present invention, or its tissue or cells derived therefrom can be used for assaying a specific activity using the homogenate of brain, liver, kidney, etc., or screening by assaying the activity or production amount of a particular product. The non-human animal deficient in apelin gene expression of the present invention, or its tissue or cells derived therefrom can also be used for screening by administering a test compound to the non-human animal deficient in apelin gene expression of the present invention, or its tissue or cells derived therefrom and assaying activities, etc.

The APJ gene-transgenic non-human animal (hereinafter, sometimes referred to as transgenic non-human animal) is, for example, a non-human animal produced by genetic engineering through microinjection of the APJ gene or its mutant DNA into fertilized eggs of a mammal. Animal is suitably a non-human mammal (e.g., rat, mouse, rabbit, sheep, swine, bovine, cat, dog, simian, etc.) etc. (hereinafter, sometimes merely referred to as animal), particularly preferably, mouse, rabbit, etc.

To transfer the APJ gene or its mutant DNA to a target animal, it is generally advantageous to use in the form of a gene construct ligated downstream a promoter capable of expressing the APJ gene or its mutant DNA in animal cells. For example, when the APJ gene or its mutant DNA is transferred to a rabbit, the gene construct ligated downstream of various promoters capable of expressing the APJ gene or its mutant DNA derived from an animal which is highly homologous thereto, is microinjected into, e.g., rabbit fertilized ova. Thus, the DNA-transferred animal (i.e., the transgenic non-human animal of the present invention) capable of producing the APJ protein, etc. in a high level can be produced. The promoter which can be used includes a virus-derived promoter and a ubiquitous expression promoter such as metallothionein, etc. An NGF gene promoter, an enolase gene promoter, and the like specifically expressed in the brain are preferably used.

The APJ gene or its mutant DNA is transfected at the fertilized egg cell stage in such a manner that the DNA is present in all of the germinal cells and somatic cells in the target animal. The fact that APJ is present in the germinal cells of the animal produced by DNA transfection means that all offspring of the produced animal will maintain the APJ protein in all of their germinal cells and somatic cells. The offspring of the animal that inherits the gene also have the APJ protein, etc. in all of their germinal cells and somatic cells.

The transgenic non-human animal bearing the APJ gene or its mutant DNA thus obtained permits the passage rearing under ordinary rearing conditions, after the individuals obtained by their crossing are proven to be knockout. Further by crossing male and female animals each carrying the target DNA, homozygous animals carrying the transgene in both loci can be obtained. The male and female animals are crossed between the animals in the way that all offsprings can be propagated and passaged to retain the DNA.

As described above, the transgenic non-human animal bearing the APJ gene or its mutant DNA thus obtained, or its tissue or cells derived therefrom can be used for screening of preventive and/or therapeutic agents for the diseases. Herein, examples of the tissues and cells derived therefrom include hypophysis, pancreas, brain, kidney, liver, gonad, thyroid, gall-bladder, bone marrow, adrenal gland, skin, muscle, lung, gastrointestinal tract, blood vessel, heart, etc., or cells derived therefrom, and the like. The transgenic non-human animal of the present invention, or its tissue or cells derived therefrom can be used for screening by assaying a particular activity using the homogenate of brain, liver, kidney, etc., or by assaying the activity or production level of a particular product. The transgenic non-human animal bearing the APJ gene or its mutant DNA of the present invention, or its tissue or cells derived therefrom can also be used for screening by administering a test compound to the tissues or cells and assaying the activity, etc.

Throughout the specification and drawings, where bases, amino acids, etc. are shown by their codes, these codes are denoted in accordance with the IUPAC-IUB Commission on Biochemical Nomenclature or by the common codes in the art, examples of which are given below. For amino acids which may have the optical isomers, L form is presented unless otherwise indicated.
- DNA :: deoxyribonucleic acid
- cDNA :: complementary deoxyribonucleic acid
- A:: : adenine
- T:: : thymine
- G:: : guanine
- C:: cytosine
- Y:: thymine or cytosine
- N:: thymine, cytosine, adenine or guanine
- R:: adenine or guanine
- M:: cytosine or adenine
- W :: thymine or adenine
- S:: cytosine or guanine
- RNA :: ribonucleic acid
- mRNA :: messenger ribonucleic acid
- ATP:: adenosine triphosphate
- EDTA :: ethylenediaminetetraacetic acid
- SDS:: sodium dodecyl sulfate
- TFA:: trifluoroacetic acid
- EIA:: enzyme immunoassay
- Gly or G:: glycine
- Ala or A :: alanine
- Val or V :: valine
- Leu or L:: leucine
- Ile or I:: isoleucine
- Ser or S :: serine
- Thr or T :: threonine
- Cys or C:: cysteine
- Met or M:: methionine
- Glu or E:: glutamic acid
- Asp or D:: aspartic acid
- Lys or K:: lysine
- Arg or R:: arginine
- His or H:: histidine
- Phe or F :: phenylalanine
- Tyr or Y :: tyrosine
- Trp or W:: tryptophan
- Pro or P :: proline
- Asn or N:: asparagine
- Gln or Q:: glutamine
- pGlu:: pyroglutamic acid
- Bom :: benzyloxymethyl
- PAM :: phenylacetamidomethyl

Substituents, protecting groups and reagents frequently used in the specification are presented by the codes below.
- Tos :: p-toluenesulfonyl
- HONB :: 1-hydroxy-5-norbornene-2,3-dicarboxyimide
- Bzl:: benzyl
- Z :: benzyloxycarbonyl
- Br-Z :: 2-bromobenzyl oxycarbonyl
- Cl-Z :: 2-chlorobenzyloxycarbonyl
- Boc :: t-butoxycarbonyl
- HOBt :: 1-hydroxybenztriazole
- DCC :: N,N'-dicyclohexylcarbodiimide
- Fmoc :: N-9-fluorenyl methoxycarbonyl
- DNP :: dinitrophenol
- Bum :: t-butoxymethyl
- Trt :: trityl

The sequence identification numbers in the sequence listing of the specification indicate the following sequences.
[SEQ ID NO: 1]
   This represents the full-length amino acid sequence of G-protein coupled receptor protein encoded by human G-protein coupled receptor protein (APJ) cDNA.
[SEQ ID NO: 2]
   This represents the full-length nucleotide sequence of human G-protein coupled receptor protein (APJ) cDNA.
[SEQ ID NO: 3]
   This represents the amino acid sequence of mouse apelin (precursor).
[SEQ ID NO: 4]
   This represents the nucleotide sequence of cDNA encoding mouse apelin (precursor).
[SEQ ID NO: 5]
   This represents the amino acid sequence of rat apelin (precursor).
[SEQ ID NO: 6]
   This represents the nucleotide sequence of cDNA encoding rat apelin (precursor).
[SEQ ID NO: 7]
   This represents the amino acid sequence of human apelin (precursor).
[SEQ ID NO: 8]
   This represents the nucleotide sequence of cDNA encoding human apelin (precursor).
[SEQ ID NO: 9]
   This represents the amino acid sequence of bovine apelin (precursor).
[SEQ ID NO: 10]
   This represents the nucleotide sequence of cDNA encoding bovine apelin (precursor).
[SEQ ID NO: 11]
   This represents the 67th to 75th amino acid sequence of the amino acid sequence of human apelin (precursor).
[SEQ ID NO: 12]
   This represents the 65th to 77th amino acid sequence of the amino acid sequence of human apelin (precursor) (apelin-13; human apelin-13, mouse apelin-13, rat apelin-13, bovine apelin-13).
[SEQ ID NO: 13]
   This represents the 42nd to 77th amino acid sequence of the amino acid sequence of human apelin (precursor) (human apelin-36).
[SEQ ID NO: 14]
   This represents the 42nd to 77th amino acid sequence of the amino acid sequence of mouse apelin (precursor) (mouse apelin-36, rat apelin-36).
[SEQ ID NO: 15]
   This represents the 42nd to 77th amino acid sequence of the amino acid sequence of bovine apelin (precursor) (bovine apelin-36).
[SEQ ID NO: 16]
   This represents the 67th to 77th amino acid sequence of the amino acid sequence of human apelin (precursor).
[SEQ ID NO: 17]
   This represents the 65th to 75th amino acid sequence of the amino acid sequence of human apelin (precursor).
[SEQ ID NO: 18]
   This represents the 64th to 77th amino acid sequence of the amino acid sequence of human apelin (precursor).
[SEQ ID NO: 19]
   This represents the 63rd to 77th amino acid sequence of the amino acid sequence of human apelin (precursor).
[SEQ ID NO: 20]
   This represents the 62nd to 77th amino acid sequence of the amino acid sequence of human apelin (precursor).
[SEQ ID NO: 21]
   This represents the 61st to 77th amino acid sequence of the amino acid sequence of human apelin (precursor).
[SEQ ID NO: 22]
   This represents the 60th to 77th amino acid sequence of the amino acid sequence of human apelin (precursor).
[SEQ ID NO: 23]
   This represents the 66th to 77th amino acid sequence of the amino acid sequence of human apelin (precursor).
[SEQ ID NO: 24]
   This represents the 65th to 76th amino acid sequence of the amino acid sequence of human apelin (precursor).

### EXAMPLES

Hereinafter the present invention will be described in detail with reference to EXAMPLES but the scope of the invention is not deemed to be limited thereto.

### EXAMPLE 1

### Decreased expression of mRNA for APJ in Brodmann area 10 from patients with depression and cocaine abusers

The expression levels of mRNA for APJ in the RNA samples derived from male patients with depression (16 cases), cocaine abusers (14 cases) and healthy volunteers (32 cases) who did not develop either disorder were assayed using the Affymetrix GeneChip set (HG_U133 A,HG_U133 B). Collection of the brain samples and analysis by GeneChip were performed at Gene Logic, Inc. (Santa Clara, CA). For data standardization the reliability test was conducted by Affimetrix Microarray Suit software (MAS) on each measurement value and the expression level of a sample where the expression was found positive was determined as the mean of relative values. As a result, the relative expression levels and expression frequencies were found that in the brains from healthy volunteers the expression level was 141.5 and the expression frequency was observed in 7 out of 32 cases, whereas the expression frequency was 0 out of 16 cases in the patients with depression and hence the expression level was 0, and in the cocaine abusers the expression frequency was only 1 out of 14 cases and the expression level was found to be 80.7. Consequently, the decreased expression levels and expression frequencies of APJ mRNA were detected in the patients with depression and the cocaine abusers.

### EXAMPLE 2

### Assessment of antidepressant effects of apelin

Effects of apelin on depression by its lateral ventricular administration can be assessed using the forced swimming test. Mature male C57BL/6N mice (9 weeks old, Charles River Japan, Inc.) received an incision in the skin overlying the skull under ethereal anesthesia 2 days before the test and a hole is drilled in the skull toward the left lateral ventricle using a two-step needle (TOP Corporation) (AP: 2 mm, lateral: 1 mm from bregma, according to the atlas of Paxinos and Watson (1986)). An hour prior to the test, magnets for measurement are adhered to the forelimbs of mice with a vinyl tape and an instant adhesive Aron-Alpha (Toa Gosei Co., Ltd.) and 20 minutes before the test phosphate buffered saline or apelin (0.3, 1 or 3 nmol/mouse) is administered for about 2 seconds using a two-step needle and a 250 µL syringe. Mice are placed in a glass aquarium, which is a transparent cylinder (height, 18 cm; diameter, 11.5 cm) containing water (24 + 1°C) up to a height of 10 cm, and forced to swim for 6 minutes. The assessment is made by the duration of immobility time and struggling time during the last 4 minutes of the 6-minute testing period.

### EXAMPLE 3

### Open field test

For animal, there were used apelin gene knockout male mice (hereinafter KO) described in WO 2006/019193, EXAMPLE 2, and wild type male mice (hereinafter WT) (18 weeks old, weighing 27-34 g, n=10 per group) obtained by mating the KO mice with C57BL/6J mice (CLEA Japan, Inc.). This test was carried out in a sound-isolated room (room temperature of 25°C) set to an illuminance level of 180 lux. Prior to testing, each mouse was allowed to acclimate for an hour or more in the chamber. Behavioral observation was made using an open field apparatus. This apparatus was constructed of a gray vinyl chloride-made plate on which a plastic cylinder covered with black tape was placed, and had a 50 cm height and a 60 cm diameter at the bottom. The locomotion area on the bottom of the plate was divided into 19 squares of almost equal area. When a mouse was moved from one square to an adjacent square, namely, all four limbs completely entered the adjacent square (which is defined as full movement to the adjacent square), the locomotor activity was scored as score 1. The moment when a mouse was placed on the centermost square at the bottom of the apparatus was made 0 second (which is referred to as the trial start) and a time period required to completely move from the centermost square to an adjacent square (hereinafter, a latent period) was measured using a stopwatch. Furthermore, the total score number of locomotor activity for 5 minutes from the trial start (hereinafter the total locomotor activity) was calculated. Student's t test was used to assay differences between the mean values.

The results are shown in FIG. 1.

As shown in FIG. 1A, the latent period was significantly extended in KO as compared to WT. Further, as shown in FIG. 1B, the total locomotor activity was significantly reduced in KO when compared to WT.

### EXAMPLE 4

### Tail suspension test

The same animals as in EXAMPLE 3 were used at least one week after the end of the test in EXAMPLE 3. This test was conducted in a sound-isolated room (room temperature of 25°C). Prior to testing, each mouse was allowed to acclimate for an hour or more in the chamber. For the tail suspension test apparatus and analysis system, MicroAct for Tail-Suspension Ver. 4.1 available from Neuroscience Idea was used. The apparatus has a compartment to suspend animal, from the ceiling of which a hook connected to a weight sensor is hung. A site of about 2 cm from the tail tip of animal was taped and the tape was pierced with the hook to suspend the animal for 10 minutes. The mobility of animal was detected with the weight sensor. When the state where a weight change was less than 10% of the body weight continued for one second or longer, it was judged as the immobility state and the sum of the immobility states for 10 minutes was considered as the immobility time. Student's t test was used for statistical analysis of the measurement values.

The results are shown in FIG. 2.

As shown in FIG. 2, the immobility time was significantly extended in KO as compared to WT.

### INDUSTRIAL APPLICABILITY

According to the present invention, excellent preventive/therapeutic agents for mood disorders or drug dependence can be provided. Furthermore, according to the present invention, the function of apelin and its receptor APJ in the central nervous system can be elucidated and a novel use mediated by these functions can be provided.

## Claims

1. A preventive/therapeutic agent for mood disorder or drug dependence comprising a substance promoting the function of an apelin receptor.

2. The agent according to claim 1, wherein the apelin receptor is a protein comprising the same or substantially the same amino acid sequence as the amino acid sequence represented by SEQ ID NO: 1.

3. The agent according to claim 1, wherein the apelin receptor is a protein comprising the amino acid sequence represented by SEQ ID NO: 1.

4. The agent according to claim 1, wherein the substance promoting the function of an apelin receptor is an agonist of the apelin receptor.

5. The agent according to claim 1, wherein the substance promoting the function of an apelin receptor is (a) apelin, (b) an apelin derivative having an activity at least equivalent to that of apelin or (c) a low molecular synthetic compound promoting the binding of apelin and its receptor.

6. The agent according to claim 5, wherein apelin is a polypeptide comprising the same or substantially the same amino acid sequence as the amino acid sequence represented by SEQ ID NO: 11.

7. The agent according to claim 5, wherein apelin is a polypeptide having the amino acid sequence represented by SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24.

8. The agent according to claim 5, wherein apelin is a polypeptide consisting of the amino acid sequence represented by SEQ ID NO: 12, SEQ ID NO: 13, SEQ ID NO: 19 or SEQ ID NO: 23.

9. The agent according to claim 1, which is a preventive/therapeutic agent for depression or anxiety.

10. A method for preventing/treating mood disorder or drug dependence, which comprises administering to a mammal an effective dose of a substance promoting the function of an apelin receptor.

11. A method for preventing/treating mood disorder or drug dependence, which comprises promoting the function of an apelin receptor.

12. Use of a substance promoting the function of an apelin receptor to manufacture a preventive/therapeutic agent for mood disorder or drug dependence.

13. The method according to claim 10 or 11, wherein the apelin receptor is a protein comprising the same or substantially the same amino acid sequence as the amino acid sequence represented by SEQ ID NO: 1.

14. The method according to claim 10 or 11, wherein the apelin receptor is a protein comprising the amino acid sequence represented by SEQ ID NO: 1.

15. The method according to claim 10 or 11, which comprises using an agonist of an apelin receptor.

16. The method according to claim 10 or 11, which comprises using (a) apelin, (b) an apelin derivative having an activity at least equivalent to that of apelin or (c) a low molecular synthetic compound promoting the binding of apelin and its receptor.

17. The method according to claim 10 or 11, wherein apelin is a polypeptide comprising the same or substantially the same amino acid sequence as the amino acid sequence represented by SEQ ID NO: 11.

18. The method according to claim 10 or 11, wherein apelin is a polypeptide having the amino acid sequence represented by SEQ ID NO: 3, SEQ ID NO: 5, SEQ ID NO: 7, SEQ ID NO: 9, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24.

19. The method according to claim 10 or 11, wherein apelin is a polypeptide consisting of the amino acid sequence represented by SEQ ID NO: 12, SEQ ID NO: 13, SEQ ID NO: 19 or SEQ ID NO: 23.

20. The method according to claim 10 or 11, which is a method for preventing/treating depression or anxiety.

21. A method for screening of a preventive/therapeutic agent for mood disorder or drug dependence, which comprises using apelin and/or an apelin receptor.

22. The method for screening according to claim 21, which comprises using a non-human animal deficient in gene expression of apelin.

23. The method for screening according to claim 21, which comprises using a transgenic non-human animal carrying an exogenous gene for an apelin receptor or its mutant DNA.

24. A kit for screening of an agent for treating mood disorder or drug dependence comprising apelin and/or an apelin receptor.


Sequence Listing

<110> Takeda Pharmaceutical Company Limited

<120> Novel Application of Apelin

<130> G08-0005

<140> PCT/JP2006/325703
<141> 2006-12-19

<150> JP2005-367165
<151> 2005-12-20

<150> JP2006-264946
<151> 2006-09-28

<160> 24

<210> 1
<211> 380
<212> PRT
<213> Homo sapiens

<400> 1
Met Glu Glu Gly Gly Asp Phe Asp Asn Tyr Tyr Gly Ala Asp Asn Gln
                5                   10                  15
Ser Glu Cys Glu Tyr Thr Asp Trp Lys Ser Ser Gly Ala Leu Ile Pro
            20                  25                  30
Ala Ile Tyr Met Leu Val Phe Leu Leu Gly Thr Thr Gly Asn Gly Leu
        35                  40                  45

Val Leu Trp Thr Val Phe Arg Ser Ser Arg Glu Lys Arg Arg Ser Ala
    50                  55                  60
Asp Ile Phe Ile Ala Ser Leu Ala Val Ala Asp Leu Thr Phe Val Val
65                  70                  75                  80
Thr Leu Pro Leu Trp Ala Thr Tyr Thr Tyr Arg Asp Tyr Asp Trp Pro
                85                  90                  95
Phe Gly Thr Phe Phe Cys Lys Leu Ser Ser Tyr Leu Ile Phe Val Asn
            100                 105                 110
Met Tyr Ala Ser Val Phe Cys Leu Thr Gly Leu Ser Phe Asp Arg Tyr
        115                 120                 125
Leu Ala Ile Val Arg Pro Val Ala Asn Ala Arg Leu Arg Leu Arg Val
    130                 135                 140
Ser Gly Ala Val Ala Thr Ala Val Leu Trp Val Leu Ala Ala Leu Leu
145                 150                 155                 160
Ala Met Pro Val Met Val Leu Arg Thr Thr Gly Asp Leu Glu Asn Thr
                165                 170                 175
Thr Lys Val Gln Cys Tyr Met Asp Tyr Ser Met Val Ala Thr Val Ser
            180                 185                 190
Ser Glu Trp Ala Trp Glu Val Gly Leu Gly Val Ser Ser Thr Thr Val
        195                 200                 205
Gly Phe Val Val Pro Phe Thr Ile Met Leu Thr Cys Tyr Phe Phe Ile
    210                 215                 220
Ala Gln Thr Ile Ala Gly His Phe Arg Lys Glu Arg Ile Glu Gly Leu
225                 230                 235                 240
Arg Lys Arg Arg Arg Leu Leu Ser Ile Ile Val Val Leu Val Val Thr
                245                 250                 255
Phe Ala Leu Cys Trp Met Pro Tyr His Leu Val Lys Thr Leu Tyr Met
            260                 265                 270
Leu Gly Ser Leu Leu His Trp Pro Cys Asp Phe Asp Leu Phe Leu Met
        275                 280                 285
Asn Ile Phe Pro Tyr Cys Thr Cys Ile Ser Tyr Val Asn Ser Cys Leu

290                 295                 300

Asn Pro Phe Leu Tyr Ala Phe Phe Asp Pro Arg Phe Arg Gln Ala Cys

305                 310                 315                 320

Thr Ser Met Leu Cys Cys Gly Gln Ser Arg Cys Ala Gly Thr Ser His

                325                 330                 335

Ser Ser Ser Gly Glu Lys Ser Ala Ser Tyr Ser Ser Gly His Ser Gln

            340                 345                 350

Gly Pro Gly Pro Asn Met Gly Lys Gly Gly Glu Gln Met His Glu Lys

        355                 360                 365

Ser Ile Pro Tyr Ser Gln Glu Thr Leu Val Val Asp

    370                 375                 380

<210> 2

<211> 1140

<212> DNA

<213> Homo sapiens

<400> 2

atggaggaag gtggtgattt tgacaactac tatggggcag acaaccagtc tgagtgtgag      60

tacacagact ggaaatcctc gggggccctc atccctgcca tctacatgtt ggtcttcctc     120

ctgggcacca cgggaaacgg tctggtgctc tggaccgtgt ttcggagcag ccgggagaag     180

aggcgctcag ctgatatctt cattgctagc ctggcggtgg ctgacctgac cttcgtggtg     240

acgctgcccc tgtgggctac ctacacgtac cgggactatg actggccctt tgggaccttc     300

ttctgcaagc tcagcagcta cctcatcttc gtcaacatgt acgccagcgt cttctgcctc     360

accggcctca gcttcgaccg ctacctggcc atcgtgaggc cagtggccaa tgctcggctg     420

aggctgcggg tcagcggggc cgtggccacg gcagttcttt gggtgctggc cgccctcctg     480

gccatgcctg tcatggtgtt acgcaccacc ggggacttgg agaacaccac taaggtgcag     540

tgctacatgg actactccat ggtggccact gtgagctcag agtgggcctg ggaggtgggc     600

cttggggtct cgtccaccac cgtgggcttt gtggtgccct tcaccatcat gctgacctgt     660

tacttcttca tcgcccaaac catcgctggc cacttccgca aggaacgcat cgagggcctg     720

cggaagcggc gccggctgct cagcatcatc gtggtgctgg tggtgacctt tgccctgtgc     780

tggatgccct accacctggt gaagacgctg tacatgctgg gcagcctgct gcactggccc 840
tgtgactttg acctcttcct catgaacatc ttcccctact gcacctgcat cagctacgtc 900
aacagctgcc tcaacccctt cctctatgcc tttttcgacc cccgcttccg ccaggcctgc 960
acctccatgc tctgctgtgg ccagagcagg tgcgcaggca cctcccacag cagcagtggg 1020
gagaagtcag ccagctactc ttcggggcac agccaggggc ccggccccaa catgggcaag 1080
ggtggagaac agatgcacga gaaatccatc ccctacagcc aggagaccct tgtggttgac 1140

<210> 3
<211> 77
<212> PRT
<213> Mus musculus

<400> 3
Met Asn Leu Arg Leu Cys Val Gln Ala Leu Leu Leu Leu Trp Leu Ser
                5                   10                  15
Leu Thr Ala Val Cys Gly Val Pro Leu Met Leu Pro Pro Asp Gly Thr
            20                  25                  30
Gly Leu Glu Glu Gly Ser Met Arg Tyr Leu Val Lys Pro Arg Thr Ser
        35                  40                  45
Arg Thr Gly Pro Gly Ala Trp Gln Gly Gly Arg Arg Lys Phe Arg Arg
    50                  55                  60
Gln Arg Pro Arg Leu Ser His Lys Gly Pro Met Pro Phe
65                  70                  75

<210> 4
<211> 231
<212> DNA
<213> Mus musculus

<400> 4
atgaatctga ggctctgcgt gcaggcgctg ctgctgctct ggctctcctt gactgcagtt 60

tgtggagtgc cactgatgtt gcctccagat ggaacaggac tagaagaagg aagcatgcgc 120

tacctggtga agcccagaac ttcgaggact ggaccaggag cctggcaggg aggcaggagg 180

aaatttcgca gacagcgccc ccggctctcc cataagggcc ccatgccttt c 231

<210> 5

<211> 77

<212> PRT

<213> Rattus norvegicus

<400> 5

Met Asn Leu Ser Phe Cys Val Gln Ala Leu Leu Leu Leu Trp Leu Ser

                5                   10                  15

Leu Thr Ala Val Cys Gly Val Pro Leu Met Leu Pro Pro Asp Gly Lys

            20                  25                  30

Gly Leu Glu Glu Gly Asn Met Arg Tyr Leu Val Lys Pro Arg Thr Ser

        35                  40                  45

Arg Thr Gly Pro Gly Ala Trp Gln Gly Gly Arg Arg Lys Phe Arg Arg

    50                  55                  60

Gln Arg Pro Arg Leu Ser His Lys Gly Pro Met Pro Phe

65                  70                  75

<210> 6

<211> 231

<212> DNA

<213> Rattus norvegicus

<400> 6

atgaatctga gtttctgcgt gcaggcgctg ctgctgctct ggctctcctt gactgccgtg 60

tgtggagtgc cactgatgct gcctccagat gggaaagggc tagaagaagg caacatgcgc 120

tacctggtga agcccagaac ttcgaggact ggaccagggg cctggcaggg aggcaggagg 180

aaatttcgca gacagcggcc ccgtctctcc cataagggac ccatgccttt c 231

<210> 7

<211> 77

<212> PRT

<213> Homo sapiens

<400> 7

Met Asn Leu Arg Leu Cys Val Gln Ala Leu Leu Leu Leu Trp Leu Ser

                5                   10                  15

Leu Thr Ala Val Cys Gly Gly Ser Leu Met Pro Leu Pro Asp Gly Asn

            20                  25                  30

Gly Leu Glu Asp Gly Asn Val Arg His Leu Val Gln Pro Arg Gly Ser

        35                  40                  45

Arg Asn Gly Pro Gly Pro Trp Gln Gly Gly Arg Arg Lys Phe Arg Arg

    50                  55                  60

Gln Arg Pro Arg Leu Ser His Lys Gly Pro Met Pro Phe

65                  70                  75

<210> 8

<211> 231

<212> DNA

<213> Homo sapiens

<400> 8

atgaatctgc ggctctgcgt gcaggcgctc ctgctgctct ggctctcctt gaccgcggtg 60

tgtggagggt ccctgatgcc gcttcccgat gggaatgggc tggaagacgg caatgtccgc 120

cacctggtgc agcccagagg gtcaaggaat gggccagggc cctggcaggg aggtcggagg 180

aaattccgcc gccagcggcc ccgcctctcc cataagggac ccatgccttt c 231

<210> 9

<211> 77

<212> PRT
<213> Bos taurus

<400> 9
Met Asn Leu Arg Arg Cys Val Gln Ala Leu Leu Leu Leu Trp Leu Cys
                5                   10                  15
Leu Ser Ala Val Cys Gly Gly Pro Leu Leu Gln Thr Ser Asp Gly Lys
            20                  25                  30
Glu Met Glu Glu Gly Thr Ile Arg Tyr Leu Val Gln Pro Arg Gly Pro
        35                  40                  45
Arg Ser Gly Pro Gly Pro Trp Gln Gly Gly Arg Arg Lys Phe Arg Arg
    50                  55                  60
Gln Arg Pro Arg Leu Ser His Lys Gly Pro Met Pro Phe
65                  70                  75

<210> 10
<211> 231
<212> DNA
<213> Bos taurus

<400> 10
atgaatctgc ggcgctgcgt gcaggcgctc ctgctgctct ggctctgcct gagcgcggtg      60
tgcggaggac ccctgctgca gacttctgac gggaaggaga tggaagaagg caccatccga     120
tacctggtgc agcccagggg gccgaggagc ggcccaggcc cctggcaggg aggtcggagg     180
aagttccggc gccagcggcc acgcctctcc cacaagggtc ccatgccttt c              231

<210> 11
<211> 9
<212> PRT
<213> Homo sapiens

<400> 11

Pro Arg Leu Ser His Lys Gly Pro Met

                5

<210> 12

<211> 13

<212> PRT

<213> Homo sapiens

<400> 12

Gln Arg Pro Arg Leu Ser His Lys Gly Pro Met Pro Phe

                5                   10

<210> 13

<211> 36

<212> PRT

<213> Homo sapiens

<400> 13

Leu Val Gln Pro Arg Gly Ser Arg Asn Gly Pro Gly Pro Trp Gln Gly

                5                   10                  15

Gly Arg Arg Lys Phe Arg Arg Gln Arg Pro Arg Leu Ser His Lys Gly

            20                  25                  30

Pro Met Pro Phe

        35

<210> 14

<211> 36

<212> PRT

<213> Mus musculus

<400> 14

Leu Val Lys Pro Arg Thr Ser Arg Thr Gly Pro Gly Ala Trp Gln Gly

                5                   10                  15

Gly Arg Arg Lys Phe Arg Arg Gln Arg Pro Arg Leu Ser His Lys Gly

            20                  25                  30

Pro Met Pro Phe

        35

<210> 15

<211> 36

<212> PRT

<213> Bos taurus

<400> 15

Leu Val Gln Pro Arg Gly Pro Arg Ser Gly Pro Gly Pro Trp Gln Gly

                5                   10                  15

Gly Arg Arg Lys Phe Arg Arg Gln Arg Pro Arg Leu Ser His Lys Gly

            20                  25                  30

Pro Met Pro Phe

        35

<210> 16

<211> 11

<212> PRT

<213> Homo sapiens

<400> 16

Pro Arg Leu Ser His Lys Gly Pro Met Pro Phe

                5                   10

<210> 17

<211> 11
<212> PRT
<213> Homo sapiens

<400> 17
Gln Arg Pro Arg Leu Ser His Lys Gly Pro Met
                5                   10

<210> 18
<211> 14
<212> PRT
<213> Homo sapiens

<400> 18
Arg Gln Arg Pro Arg Leu Ser His Lys Gly Pro Met Pro Phe
                5                   10

<210> 19
<211> 15
<212> PRT
<213> Homo sapiens

<400> 19
Arg Arg Gln Arg Pro Arg Leu Ser His Lys Gly Pro Met Pro Phe
                5                   10                  15

<210> 20
<211> 16
<212> PRT
<213> Homo sapiens

<400> 20

Phe Arg Arg Gln Arg Pro Arg Leu Ser His Lys Gly Pro Met Pro Phe

                    5                   10                  15

<210> 21

<211> 17

<212> PRT

<213> Homo sapiens

<400> 21

Lys Phe Arg Arg Gln Arg Pro Arg Leu Ser His Lys Gly Pro Met Pro

                    5                   10                  15

Phe

<210> 22

<211> 18

<212> PRT

<213> Homo sapiens

<400> 22

Arg Lys Phe Arg Arg Gln Arg Pro Arg Leu Ser His Lys Gly Pro Met

                    5                   10                  15

Pro Phe

<210> 23

<211> 12

<212> PRT

<213> Homo sapiens

<400> 23

Arg Pro Arg Leu Ser His Lys Gly Pro Met Pro Phe

                5                   10

<210> 24

<211> 12

<212> PRT

<213> Homo sapiens

<400> 24

Gln Arg Pro Arg Leu Ser His Lys Gly Pro Met Pro

                5                   10